# EUROPEAN PATENT APPLICATION

(11) **EP 2 316 496 A2**
(43) Date of publication of application: **04.05.2011**
(21) Application number: 10251728.1
(22) Date of filing: 04.10.2010
(51) Int. Cl.: A61L 17/04, A61L 17/14

(54) **Coatings that enhance resistance to abrasion**

(30) Priority: 05.10.2009 US 248610 P; 24.09.2010 US 889504
(71) Applicant: Tyco Healthcare Group LP, New Haven, CT 06511 (US)
(72) Inventor: Abuzaina, Ferrass, Shelton, CT 06484 (US)
(74) Representative: Soames, Candida Jane

(57) **Abstract**

The present disclosure provides coatings for filaments that enhance the resistance of a filament to cuts and/or abrasions. Depending upon the material utilized to form the filament to be coated, the appropriate coating in the appropriate amount may be selected to coat the filament thereby imparting resistance to abrasions and cuts to the filament. The resulting filament may be utilized to form filamentous devices including sutures and meshes.

## Description

### CROSS-REFERENCE TO RELATED APPLICATIONS

This application claims the benefit of, and priority to, U.S. Provisional Application No. 61/248,610, filed on October 5, 2009, the entire disclosure of which is incorporated by reference herein.

### BACKGROUND

The present disclosure relates to coatings capable of enhancing the resistance of medical devices, in embodiments sutures or devices formed from filaments, to abrasions and cuts which may form during application of the device to a site of injury and/or in situ.

Sutures, including coated sutures, are within the purview of those skilled in the art. In use, sutures may be exposed to sharp edges, such as broken or fractured bone edges, screws, plate edges, anchor eyelets and the like, as well as manipulated with knot pushers that could have sharp or abrading edges.

Filaments used to form sutures may similarly be used to form other devices, including meshes. These medical devices may also become abraded or cut upon contact with sharp edges. Accordingly, medical devices having a high resistance to abrasion and cuts for use in surgical procedures remain desirable.

### SUMMARY

The present disclosure provides medical devices, in embodiments sutures, and coatings suitable for use thereon which enhance the abrasion resistance of the device. In embodiments, a medical device of the present disclosure may include at least one non-bioabsorbable filament including a polymer including a polyester, and a coating including a two part silicone on at least a portion of a surface of the non-bioabsorbable filament, wherein the two part silicone is at a concentration of from about 1% to about 30% by weight in solution.

In other embodiments, a medical device of the present disclosure may include at least one non-bioabsorbable filament including a polymer including a polyester, and a coating comprising a polybutylene adipate on at least a portion of a surface of the non-bioabsorbable filament, wherein the polybutylene adipate is at a concentration of from about 1% to about 10% by weight in solution.

In yet other embodiments, a medical device of the present disclosure may include at least one non-bioabsorbable filament including a polymer including a polyester, and a coating including a polyethylene wax on at least a portion of a surface of the non-bioabsorbable filament, wherein the polyethylene wax is at a concentration of from about 10% to about 30% by weight in solution.

### BRIEF DESCRIPTION OF THE DRAWINGS

The foregoing objects and advantages of the disclosure will become more apparent from the reading of the following description in connection with the accompanying drawings, in which:

FIG. 1 is a graph of cycles to failure for biodegradable sutures coated with four different coatings of the disclosure;

FIG. 2 is a graph of cycles to failure for non-bioabsorbable sutures coated with four different coatings of the disclosure;

FIG. 3 is a graph of results for three handling tests on biodegradable sutures coated with four different coatings of the disclosure; and

FIG. 4 is a graph of results for three handling tests on non-bioabsorbable sutures coated with four different coatings of the disclosure.

### DETAILED DESCRIPTION

The present disclosure is directed to medical devices formed from coated filaments. The devices may be sutures including a single filament (monofilament), or multiple filaments (multifilament). The coatings applied to the filaments of the present disclosure increase the resistance of the filaments to abrasions and/or cuts and/or increase handling characteristics of the suture. In addition to sutures, in embodiments, the resulting filaments may be used to form other medical devices such as surgical mesh.

The filaments may be formed from any biocompatible material that has suitable physical properties for the intended use of the filament. Methods for preparing compositions suitable for filaments as well as techniques for making filaments from such compositions are within the purview of those skilled in the art.

The filaments of the present disclosure may be formed from biodegradable or non-biodegradable materials. The term "biodegradable" as used herein is defined to include both bioabsorbable and bioresorbable materials. By biodegradable, it is meant that the materials decompose, or lose structural integrity under body conditions (e.g., enzymatic degradation or hydrolysis) or are broken down (physically or chemically) under physiologic conditions in the body such that the degradation products are excretable or absorbable by the body. Suitable bioabsorbable materials, sometimes referred to herein as biodegradable materials, include, but are not limited to, natural collagenous materials or synthetic resins including those derived from alkylene carbonates such as trimethylene carbonate, tetramethylene carbonate, and the like; caprolactone; dioxanone; poly(glycolic acid); poly(lactic acid); homopolymers thereof, copolymers thereof, and combinations thereof.

In embodiments biodegradable filaments may be utilized to form sutures. Bioegradable sutures of the present disclosure may be short term biodegradable sutures or long term biodegradable sutures. The classification short term biodegradable sutures generally refers to surgical sutures which retain about 20 percent of their original strength at about three weeks after implantation, with the suture mass being completely degraded in the body within about 60 to about 90 days post implantation. Examples of commercially available short term degradable multifilament sutures include VICRYL® from Ethicon, Inc. (Somerville, N.J.), and POLYSORB™ from Tyco Healthcare Group LP, d/b/a Covidien (North Haven, CT), hereinafter referred to as "Covidien."

In some embodiments, long term biodegradable sutures may be used to form sutures of the present disclosure. Long term biodegradable sutures include sutures which retain about 20 percent of their original strength at about six or more weeks after implantation, with the suture mass being completely degraded in the body within about 180 days post implantation. MAXON™ sutures, commercially available from Covidien, are other biodegradable synthetic monofilament sutures, which begin exhibiting mass loss at about 90 days after implantation, with the suture mass being completely degraded in the body about 180 days after implantation. MAXON™ sutures are prepared from a copolymer of glycolic acid and trimethylene carbonate.

Yet other sutures which may be utilized include BIOSYN™ sutures, commercially available from Covidien, which are biodegradable monofilament sutures made from a terpolymer of glycolide, trimethylene carbonate, and dioxanone. These sutures are stronger than braided synthetic biodegradable sutures over 4 weeks post implantation, but are completely degraded between about 90 and about 110 days post implantation. Examples of specific long term biodegradable materials include those disclosed in U.S. Patent No. 6,165,202, the entire disclosure of which is incorporated by reference herein.

In still another embodiment, a suture of the present disclosure may be made of a quaternary polymer of glycolide, trimethylene carbonate, caprolactone, and L-lactide, wherein the polymer includes from about 62% to about 72% by weight glycolide, in embodiments from about 67% to about 71 % by weight glycolide, from about 1% to about 10% by weight trimethylene carbonate, in embodiments from about 6% to about 8% by weight trimethylene carbonate, from about 12% to about 20% by weight caprolactone, in embodiments from about 15% to about 18% by weight caprolactone, and from about 1% to about 10% by weight L-lactide, in embodiments from about 6% to about 8% by weight L-lactide.

In another embodiment, a suture of the present disclosure may be made of 100% glycolide or 100% polydioxanone. In a further embodiment, a suture of the present disclosure may be made of a glycolide-L-lactide copolymer wherein the copolymer includes from about 87% to about 99% by weight glycolide, in embodiments from about 89% to about 93% by weight glycolide, and from about 4% to about 13% by weight of L-lactide, in embodiments from about 7% to about 11% by weight of L-lactide.

Some specific non-limiting examples of suitable non-bioabsorbable materials which may be utilized to form the filaments of the present disclosure include, for example, polyolefins, such as polyethylene, polypropylene, copolymers or blends of polyethylene and polypropylene; polyamides; segmented polyether-ester block copolymers; polyurethanes; polyacrylonitriles; and combinations thereof. In embodiments, silk, cotton, linen, carbon fibers, steel fibers, or other biologically acceptable non-bioabsorbable materials may be used. It should, of course, be understood that combinations of bioabsorbable and non-bioabsorbable materials may be used to form the filaments.

In embodiments, the filaments may be made of polyethylene terephthalate. Polyethylene terephthalate is a non-bioabsorbable thermoplastic polyester formed by the esterification from ethylene glycol and terephthalic acid. Its advantageous properties include high tensile strength, high resistance to stretching under both wet and dry conditions, and good resistance to degradation by chemical bleaches and to abrasion. Examples of suitable polyethylene terephthalate sutures that may be utilized include TI•CRON™ sutures commercially available from Covidien.

The filaments may be made using any technique within the purview of those skilled in the art, such as, for example, extrusion, molding, and/or gel spinning. In embodiments, the strands may be extruded through an extruder unit of a conventional type, such as those disclosed in U.S. Patent Nos. 6,063,105, 6,203,564, and 6,235,869, the entire disclosures of each of which are incorporated by reference herein. In embodiments, filaments of dissimilar materials may be extruded separately and subsequently brought together into a group to form a yarn, or the strands can be extruded in a side-by-side fashion and collected together to immediately form a yarn. The number of filaments used per yam may depend on a number of factors, including the desired final size of the yam and the ultimate multi-filament article being produced. For example, with respect to sutures, size is established according to United States Pharmacopoeia ("USP") standards.

Once formed, a plurality of the filaments may then be braided, twisted, entangled, intertwined, or arranged in some other multifilament configuration. The braiding can be done by any method within the purview of those skilled in the art. For example, braid constructions for sutures and other medical devices are described in U.S. Patent Nos. 5,019,093; 5,059,213; 5,133,738; 5,181,923; 5,226,912; 5,261,886; 5,306,289; 5,318,575; 5,370,031; 5,383,387; 5,662,682; and 5,667,528; the contents of each of which are incorporated by reference herein.

Filaments, braids, or yarns formed therefrom may be used in the fabrication, in whole or in part, of a variety of medical devices for example, include: sutures; tapes; gauze; wound dressings; meshes; grafts (e.g., fabrics and/or tubes); rings; prosthetic soft tissue (e.g., tendons and/or ligaments); growth matrices; drug delivery devices; and other implantable medical devices. As used herein, an implantable medical device includes any device which can be implanted in an animal for medical purposes. The filaments, braids, or yarns may be braided, knitted or woven to form the device of the present disclosure.

In accordance with the present disclosure, the filaments and/or medical devices formed therefrom are coated. Suitable coatings include: urethanes; waxes; esters; silicon-based coatings; and combinations thereof. In accordance with the present disclosure, depending upon the material utilized to form the filaments; various coatings may be applied thereto to enhance the resistance of the filaments to cuts and/or abrasion.

In embodiments, the coating materials may be combined with a solvent. The solvent used may be chosen by one skilled in the art based on a variety of factors such as, for example, the coating material, desired thickness of the coating, and construction of the device. In embodiments, the solvent may be, for example, methylene chloride, hexane, chloroform, ethanol, methanol, tetrahydrofuran (THF), methylethyl ketone(MEK), isopropanol, methylene chloride, xylene, combinations thereof, and the like. The concentration of the coating materials in any solution may be from about 1% to about 30% by weight, in embodiments, about 2% to about 20% by weight.

The coatings may include, in embodiments, thermoplastic polyurethanes (TPU) such as, for example, polyether-based TPUs and polycarbonate-based TPUs. In embodiments, suitable aliphatic polyether-based TPUs include SG-93A, SG-85A, SG-80A, and SG-60D, commercially available from Lubrizol, sold under the tradename TECOFLEX^{®}. In embodiments suitable aromatic polyether-based TPUs which may be used include TT-74A and TT-85A, commercially available from Lubrizol, sold under the tradename TECOTHANE^{®}. Suitable aliphatic polycarbonate-based TPUs include PC-3575A, PC-3585A, PC-3595A, and PC-3555D, all commercially available from Lubrizol, sold under the tradename CARBOTHANE^{®}. In embodiments, suitable polyurethanes include segmented polymers having phenylene diisocyanate as the hard segment and either polypropylene glycol adipate or polyethylene glycol-polypropylene glycol adipate as the hard segment.

The urethane-based coatings utilized in accordance with the present disclosure may exhibit various levels of hardness, as measured by durometer. In embodiments, the coating may have a durometer from about 70A to about 60D.

In embodiments, the concentration of the polyurethane coating may be about 1% by weight or about 10% by weight. More specifically, in embodiments, the concentration of an aliphatic polyether TPU in a coating solution may be from about 1% to about 10% by weight of a coating solution, in embodiments from about 2% to about 6% by weight of a coating solution, in embodiments from about 2.5% by weight to about 5% by weight of a coating solution. In embodiments, the concentration of an aromatic polyether TPU in a coating solution may be from about 1% to about 10% by weight of a coating solution, in embodiments from about 2% to about 6% by weight of a coating solution, in embodiments from about 2.5% by weight to about 5% by weight of a coating solution. In embodiments, the concentration of an aliphatic polycarbonate in a coating solution may be from about 1% to about 10% by weight of a coating solution, in embodiments from about 2% to about 6% by weight of a coating solution, in embodiments from about 2.5% by weight to about 5% by weight of a coating solution.

In embodiments, the coating may include waxes such as polyolefin waxes, for example, polyalkylene waxes, polypropylene waxes, polyethylene waxes, bees wax, and combinations thereof. The polyolefins may be aliphatic or aromatic. In embodiments, the concentration of a wax used as a coating may be from about 10% to about 30% by weight of a coating solution, in embodiments from about 15% to about 25% by weight of a coating solution, in embodiments about 20% by weight of a coating solution.

In embodiments, the coating may include polyesters such as, for example, polyesters based upon polyglycolic acid, polylactic acid, poly(lactic-co-glycolic acids), polydioxanones, polycaprolactones, polyhydroxylalkanoates, copolymers based on cyclic aliphatic esters, polyethylene terephthalate, polybutylene adipate, combinations thereof, and the like.

In embodiments, the concentration of a polyester such as polybutylene adipate (PBA) may be from about 1% by weight to about 10% by weight of a coating solution, in embodiments from about 1% to about 5% by weight of a coating solution, in embodiments about 2.5% by weight of a coating solution.

In embodiments, the coating may include silicon based coatings such as, siloxanes, polyorganosiloxanes, polydiorganosiloxanes, silanes, aminoalkylsiloxanes, cyclosiloxanes, polydimethylsiloxanes, hydrocyclosiloxanes, platinum cured silicones, combinations thereof, and the like. Suitable silicones include, for example, MED-4755, MED-4770, MED-4780, and MED-6640, all commercially available from Nusil Silicone Technology. In embodiments, a platinum cured 2-part silicone, commercially available from Nusil Silicone Technology, may be utilized.

In embodiments, the concentration of a silicone used as a coating may be from about 1% to about 30% by weight of a coating solution, in embodiments from about 5% to about 15% by weight of a coating solution, in embodiments about 10% by weight of a coating solution.

Combinations of any of the foregoing coatings may be utilized in embodiments.

The coating of the disclosure may be applied in discrete locations or, in embodiments, may be applied along the entire surface of the filament. Where applied in discrete locations, the locations may be intermittent or may be along one or more partial lengths of continuous and/or increasing and/or decreasing lengths along the filament.

The coatings described herein may be applied to a filament and/or medical device by any technique including, but not limited to, dipping, spraying, brushing, wiping, or any other appropriate technique for forming a continuous layer onto the surface of an implantable device. The particular technique used may be chosen by those skilled in the art depending upon a variety of factors such as the specific construction of the filament and the material contained in the coating.

The coated filaments and/or medical device may be dried, if necessary, using any suitable technique including, but not limited to, the use of an oven. In some embodiments, the coated filament may be dried under vacuum at a temperature of about 40° C. In some embodiments, a convection oven may be used to drive off any solvent utilized in the coating.

The coated filaments and/or medical devices of the disclosure may include bioactive agents. The term "bioactive agent," as used herein, is used in its broadest sense and includes any substance or mixture of substances that have clinical use. Consequently, bioactive agents may or may not have pharmacological activity per se, e.g., a dye. Alternatively a bioactive agent could be any agent, which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the present implant in any suitable form of matter, e.g., films, powders, liquids, gels and the like.

Examples of classes of bioactive agents, which may be utilized in accordance with the present disclosure for example, include: anti-adhesives; antimicrobials; analgesics; antipyretics; anesthetics; antiepileptics; antihistamines; anti-inflammatories; cardiovascular drugs; diagnostic agents; sympathomimetics; cholinomimetics; antimuscarinics; antispasmodics; hormones; growth factors; muscle relaxants; adrenergic neuron blockers; antineoplastics; immunogenic agents; immunosuppressants; gastrointestinal drugs; diuretics; steroids; lipids; lipopolysaccharides; polysaccharides; platelet activating drugs; clotting factors; and enzymes. It is also intended that combinations of bioactive agents may be used.

Anti-adhesive agents can be used to prevent adhesions from forming between the coated filament and the surrounding tissues to which the filament is applied. In addition, anti-adhesive agents may be used to prevent adhesions from forming between the coated filament and the packaging material. Some examples of these agents include, but are not limited to hydrophilic polymers such as poly(vinyl pyrrolidone), carboxymethyl cellulose, hyaluronic acid, polyethylene oxide, poly vinyl alcohols, and combinations thereof.

Suitable antimicrobial agents, which may be included as a bioactive agent include: triclosan, also known as 2,4,4'-trichloro-2'-hydroxydiphenyl ether; chlorhexidine and its salts, including chlorhexidine acetate, chlorhexidine gluconate, chlorhexidine hydrochloride, and chlorhexidine sulfate; silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides, such as tobramycin and gentamicin, rifampicin, bacitracin, neomycin, chloramphenicol, and miconazole; quinolones, such as oxolinic acid, norfloxacin, nalidixic acid, pefloxacin, enoxacin and ciprofloxacin; penicillins, such as oxacillin and pipracil; nonoxynol 9; fusidic acid; cephalosporins; and combinations thereof. In addition, antimicrobial proteins and peptides such as bovine lactoferrin and lactoferricin B may be included as a bioactive agent.

Other bioactive agents, which may be included as a bioactive agent include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anticonvulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins; cytotoxic drugs; chemotherapeutics, estrogens; antibacterials; antibiotics; anti-fungals; anti-virals; anticoagulants; anticonvulsants; antidepressants; antihistamines; and immunological agents.

Other examples of suitable bioactive agents, which may be included in the coating include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids, such as antisense molecules, DNA, RNA, RNAi; oligonucleotides; polynucleotides; and ribozymes.

The coated filament and/or medical device may also include, for example, biologically acceptable plasticizers, antioxidants, and/or colorants, which can be impregnated into the medical device.

The structural capabilities of the coated filaments may be tested, for example, for abrasion resistance and/or handling. Abrasion resistance may be tested by determining the number of cycles to failure using, for example, a Taber Abrasion Test or other devices for testing fatigue such as the ELECTROPULSE™ testing machine available from Instron, utilizing the manufacturer's directions. Handling may be evaluated with four tests, knot security, knot handling, knot repositioning, and knot rundown. Knot security or strength may be determined using a Tensile Testing Machine available from Instron, utilizing the manufacturer's directions. Knot handling may be determined using a wet and/or dry suture and applying a surgeon's throw on a tie board to ensure no slippage of the knot occurs during suturing. Knot repositioning and rundown are also tested on a tie board. Such devices and methods for testing abrasion resistance and handling are commonly used and within the purview of those of skill the art.

It should be noted that as referenced herein, a Polysorb ™ coated suture refers to uncoated Polysorb ™ which is then coated with one of the coatings described in the present disclosure, and where relevant, similarly for Ticron ™. Production Polysorb ™ and Ticron ™ will be referenced herein as such.

In some embodiments, a coated Polysorb ™ suture can have an abrasion resistance, sometimes referred to herein as exhibiting a fatigue, of from about 3 cycles to failure to about 610 cycles to failure, in embodiments from about 10 cycles to failure to about 500 cycles to failure. For example, Polysorb ™ coated with a polyether-based polyurethane coating may have an average fatigue of from about 11 cycles to failure to about 480 cycles to failure; a bioabsorbable suture coated with a 2-part silicone coating may have an average fatigue of from about 173 cycles to failure to about 415 cycles to failure; Polysorb ™ coated with a polyethylene wax may have an average fatigue of from about 150 cycles to failure to about 450 cycles to failure, in embodiments about 300 cycles to failure; and Polysorb ™ suture coated with a polybutylene adipate coating may have an average fatigue of from about 271 cycles to failure to about 470 cycles to failure, in embodiments about 375 cycles to failure. Comparatively, uncoated Polysorb ™ has an average cycles to failure of about 5, while production coated Polysorb ™ has an average cycles to failure of about 225.

In embodiments, a coated Ticron ™ suture may have an average fatigue of from about 1 to about 240 cycles to failure. For example a Ticron ™ suture with a 2-part silicone coating may have an average fatigue of from about 20 to about 230 cycles to failure; Ticron ™ suture with a polybutylene adipate coating may have an average fatigue of from about 74 cycles to failure to about 236 cycles to failure, in embodiments about 165 cycles to failure; Ticron ™ suture with a polyethylene wax coating may have an average fatigue of from about 67 cycles to failure to about 202 cycles to failure, in embodiments about 159 cycles to failure. Comparatively, uncoated Ticron ™ has an average cycles to failure of at least 1, while production coated Ticron ™ has an average cycles to failure of about 24.

In order that one skilled in the art may be better able to practice the compositions and methods described herein, the following examples are provided as an illustration of the coated medical devices of the present disclosure.

### EXAMPLES

### EXAMPLE 1

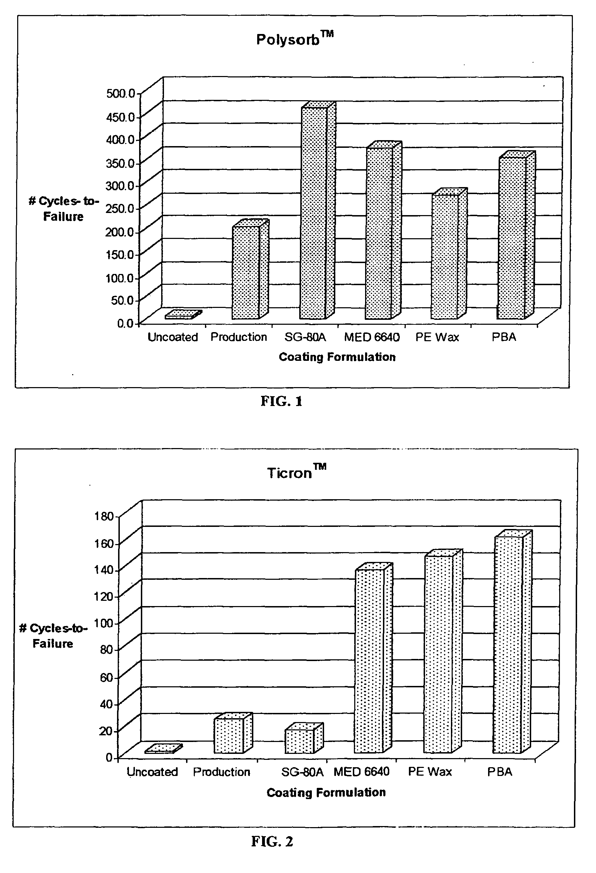

Braided absorbable glycolide-lactide copolymer sutures (POLYSORB™) were dip coated with various coatings. Abrasion resistance testing was performed using an Instron E3000 ELECTROPULSE™ testing machine. Each suture was threaded through a titanium eyelet of a HERCULON™ anchor pin then passed over two pulleys with a 1 kg weight attached to the end. In this manner the suture was rubbed against the eyelet edge in a cyclic fashion until it frayed or broke. A new anchor pin was used for each suture to prevent polishing or distortion of the pin surface. For each coating type, 10 sutures were tested. The list of coatings and solvents is provided below in Table 1. The mean number of cycles to failure for each type of coated suture on each pin is also listed for each coating. Results for uncoated, production coated, SG-80, MED-6640, PE wax and PBA coatings are also displayed in FIG. 1.

Some of the coated sutures were tested in a saline bath at 37°C. These results are also listed in Table 1.

**TABLE 1**

| Durometer (Shore Hardness) | Coating Solution (W/W%) | Coating Formulation | Solvent | Dry Mean | Dry Std Dev | Wet Mean | Wet Std. Dev. |
|---|---|---|---|---|---|---|---|
| 87A | 5 | Aliphatic polyether based TPU SG-93A | Methylene Chloride | 15.5 | 8.5 | | |
| 72A | 5 | Aliphatic polyether based TPU SG-80A | Methylene Chloride | 489.3 | 77.9 | 8.2 | 2.2 |
| 51D | 5 | Aliphatic polyether based TPU SG-60D | Chloroform | 10.7 | 2.9 | | |
| 87A | 2.5 | Aliphatic polyether based TPU SG-93A | Methylene Chloride | 17.8 | 14.1 | | |
| 77A | 2.5 | Aliphatic polyether based TPU SG-85A | | 295.4 | 123.4 | | |
| 55A | 10 | 2 Part Silicone MED- 4755 1:1 ratio | Hexane | 417.2 | 34.1 | | |
| 70A | 10 | 2 Part Silicone MED- 4770 1:1 ratio | Hexane | 353.6 | 40.8 | | |
| 80A | 10 | 2 Part Silicone MED- 4780 1:1 ratio | Hexane | 314.8 | 80.0 | | |
| | ∼10 | 440s-055 (PU) | THF | 45.9 | 23.3 | | |
| | ∼2.38 | 440s-059 (PU) | THF | 187.1 | 99.3 | | |
| 40A | 10 | 2 Part Silicone MED- 6640 1:1 ratio | Hexane (50%) Xylene (40%) | 416.7 | 20.6 | 79.3 | 22.5 |
| 84A | 5 | Aliphatic polycarbonate based TPUs PC-3585A | Methylene Chloride | 23.7 | 16.6 | | |
| 84A | 5 | Aliphatic polycarbonate based TPUs PC-3585A | Chloroform | 32.7 | 21.3 | | |
| 60D | 5 | Aliphatic polycarbonate based TPUs PC-3555D | Chloroform | 10.3 | 5.1 | | |
| 95A | 5 | Aliphatic polycarbonate based TPUs PC-3595A | Chloroform | 16.7 | 6.8 | | |
| | 20 | Poly ethylene wax | Xylene | 303.0 | 101.3 | 18.1 | 5.2 |
| | 2.5 | Polybutylene adipate (PBA) | MEK | 374.5 | 60.9 | 24.6 | 11.6 |
| 75A | 5 | Aromatic polyether based TPU TT-74A | THF | 124.6 | 91.2 | | |
| 85A | 5 | Aromatic polyether based TPU TT-85A | THF | 92.3 | 121.2 | | |
| 72A | 5 | Aliphatic polyether based TPU SG-80A | THF | 462.1 | 45.1 | | |
| 73A | 5 | Aliphatic polycarbonate based TPUs PC-3575A | THF | 92.1 | 66.1 | | |
| | | Uncoated | | 5.5 | 4.4 | 12.1 | 2.9 |
| | | Production Coated | | 225.5 | 63.8 | 12.8 | 2.3 |

Unexpectedly, as shown in Table 1 bove, the softer thermoplastic aliphatic polyether based coatings provided greater abrasion resistance. Specifically, the aliphatic polyether based TPU, SG-80A (from THERMEDICS™), provided excellent abrasion resistance. A 5% coating of the SG-80A (from THERMEDICS™) resulted in a mean number of cycles to failure of 489, and a 2.5% coating of the aliphatic polyether based TPU, SG-85A (from THERMEDICS™), resulted in a mean number of cycles to failure of 295. A 5% coating of the aliphatic polyether based TPU SG-93A (from THERMEDICS™), a 5% coating of the aliphatic polyether based TPU SG-60D (from THERMEDICS™), and a 2.5% coating of the aliphatic polyether based TPU SG-93A (from THERMEDICS™), all had a mean number of cycles to failure of less than 20. Of the aromatic polyether based TPUs (TT-74A and TT-85A) (both from THERMEDICS™) the higher durometer TT-74A provided a mean number of cycles to failure of 124 while the TT-85A provided a number of cycles to failure of 92.

By contrast, the 2-part silicone coatings provided a mean number of cycles to failure over 300, irrespective of durometer number. The PBA and the PE-wax also provided a mean number of cycles to failure of over 300. Of the aliphatic polycarbonate based TPUs tested (PC-3585A, PC-3555D, PC-3595A, and PC-3575A) (from Nusil Silicone Technology), only the PC-3575A provided a mean number of cycles to failure greater than 32.

Biodegradable sutures coated with PBA, polyethylene wax, and MED-6640 showed improved abrasion resistance over both the uncoated and production coated sutures when subject to a saline bath at 37°C.

### EXAMPLE 2

Braided polyester sutures, formed from polyethylene terephthalate (TI•CRON™ sutures), were coated with various coatings. Abrasion resistance testing was performed using an Instron E3000 ELECTROPULSE™ testing machine. Each suture was threaded through a titanium eyelet of a HERCULON™ anchor pin then passed over two pulleys with a 1 kg weight attached to the end. In this manner the suture was rubbed against the eyelet edge in a cyclic fashion until it frayed or broke. A new anchor pin was used for each suture to prevent polishing or distortion of the pin surface. For each coating type, 10 sutures were tested. The list of coatings and solvents is provided below in Table 2. The mean number of cycles on each pin prior to breakage is also listed for each coating. The list of coatings and solvents and the mean number of cycles on each pin prior to breakage for 10 sutures having the same coating is listed below in Table 2. Results for uncoated, production coated, SG-80, MED-6640, PE wax and PBA coatings are also displayed in FIG. 2.

Some of the coated sutures were tested in a saline bath at 37°C. These results are also listed in Table 2.

**TABLE 2**

| Durometer (Shore Hardness) | Coating Solution (W/W%) | Coating Formulation | Solvent | Dry Mean | Std. Dev. | Wet Mean | Std. Dev. |
|---|---|---|---|---|---|---|---|
| 80A | 10 | 2 Part Silicone MED- 4780 1:1 ratio | Hexane | 54.6 | 13.0 | | |
| 77A | 2.5 | Aliphatic polyether based TPU SG-85A | Methylene Chloride | 1.4 | 0.6 | | |
| 70A | 10 | 2 Part Silicone MED- 4770 1:1 ratio | Hexane | 37.5 | 9.3 | | |
| 55A | 10 | 2 Part Silicone MED- 4755 1:1 ratio | Hexane | 61.0 | 15.3 | | |
| 40A | 10 | 2 Part Silicone MED- 6640 | Hexane (50%) Xylene (40%) | 135.5 | 49.0 | 132.9 | 59.7 |
| | ∼10 | 440s-055 (PU) | THF | 1.1 | 0.6 | | |
| 72A | 5 | Aliphatic polyether based TPU SG-80A | Methylene Chloride | 17.3 | 6.0 | 2.7 | 0.5 |
| 87A | 2.5 | Aliphatic polyether based TPU SG-93A | Methylene Chloride | 0.5 | 0.4 | | |
| | ∼2.38 | 440s-059 (PU) | THF | 8.8 | 5.0 | | |
| 84A | 5 | Aliphatic polycarbonate based TPUs PC-3585A | Methylene Chloride | 1.5 | 1.3 | | |
| 84A | 5 | Aliphatic polycarbonate based TPUs PC-3585A | Chloroform | 1.8 | 1.0 | | |
| 60D | 5 | Aliphatic polycarbonate based TPUs PC-3555D | Chloroform | 2.2 | 1.9 | | |
| 95A | 5 | Aliphatic polycarbonate based TPUs PC-3595A | Chloroform | 1.3 | 1.1 | | |
| | 20 | Poly ethylene wax | Xylene | 158.5 | 39.6 | 47.0 | 10.2 |
| | 2.5 | Polybutylene adipate (PBA) | MEK | 166.4 | 49.4 | 63.8 | 19.2 |
| 75A | 5 | Aromatic polyether based TPU TT-74A | THF | 3.9 | 2.7 | | |
| 85A | 5 | Aromatic polyether based TPU TT-85A | THF | 1.3 | 0.9 | | |
| 72A | 5 | Aliphatic polyether based TPU SG-80A | THF | 16.2 | 9.4 | | |
| 73A | 5 | Aliphatic polycarbonate based TPUs PC-3575A | THF | 2.5 | 1.8 | | |
| | N/A | Uncoated | | 1.5 | 1.4 | 1.0 | 0.5 |
| | | Production Coated | | 23.8 | 11.7. | 113.2 | 37.2 |

In contrast to the results obtained for the biodegradable suture in Example 1, the abrasion resistance of the non-biodegradable suture of Example 2 did not improve with application of a polyurethane coating. Abrasion resistance improved with the application of 2-part silicone: a mean number of cycles to failure of 37.5 with MED-4770 (from Nusil Silicone Technology); a mean number of cycles to failure of 54.6 for MED-4780 (Nusil Silicone Technology); a mean number of cycles to failure of 61 for MED-4755 (Nusil Silicone Technology); and a mean number of cycles to failure of 135.5 for MED-6640 (Nusil Silicone Technology). The polyethylene wax and the PBA coating increased the number of cycles to failure to 158.5 and 166.4, respectively.

Ticron™ sutures coated with MED-6640 (Nusil Silicone Technology), an aliphatic polyether based TPU, SG-80A (from THERMEDICS™), a polyethylene wax, and PBA, which were subjected to a saline bath at 37°C, showed improved performance over the uncoated Ticron ™ suture. Additionally, the suture coated with MED-6640 also showed improvement over the production coated suture.

### EXAMPLE 3

In order to determine the effect of the coatings on the suture, handling tests were performed on coated and uncoated biodegradable (Polysorb ™) and non-biodegradable (Ticron™) sutures. An Instron Tensile Testing machine having a 1.5 inch foam covered mandrel was used to determine knot security. Sutures were individually tied around the mandrel using a consistent knot type. After forming the knot, the side of the suture opposite the knot on the mandrel was cut to form sample ends. The knot was then pulled to a preset load. A knot was considered secure if the knot went to knot break without slipping more than 3 mm.

Knot handling, repositioning, and rundown were measured on a standard tie board. A tie board includes a base on which two plates are perpendicularly affixed. These plates are parallel to one another on the base and are separated by a distance of at least 3 inches. Each plate contains two oppositely disposed openings, the distance between the openings on one plate being longer than that of the other plate. An elastic tube is passed through the openings on both plates to complete a loop which is then tied to secure the loop to the plates. The loop is in the general configuration of an isosceles triangle.

A surgeon's throw was performed by looping each suture around the elastic tubes of the tie board and tying them with a surgeon's throw (a half hitch with an extra loop of the free end). The outward force created by the elastic tubes of the tie board approximates the force exerted by living tissue on a suture knot. The ends were then pulled apart by hand, drawing the elastic tubes of the tie board together. The ends of the suture were then released. If no slippage occurred, additional force was applied to the bands until the throw slipped. Sutures passed the test if the throw did not slip when the suture was released.

Knot repositioning was also tested on a tie board. A single surgeon's throw of the suture around the elastic bands was performed and the suture was run down the elastic bands to approximately three inches above the tie board bands. A second single surgeon's throw (in the same direction as the first) was performed on the same suture and rundown to the first throw. The free ends of the suture were then pulled apart by hand. If the knot slipped and the loop of the suture pulled the elastic tubes of the tie board together, the knot repositioned and the test passed. To test knot rundown, i.e., the drag of the suture during repositioning, two more surgeon's throws were performed and rundown to the first surgeon's throw. Knot rundown was rated on a scale of 1 to 5, where 1 was "not acceptable" and 5 was "excellent."

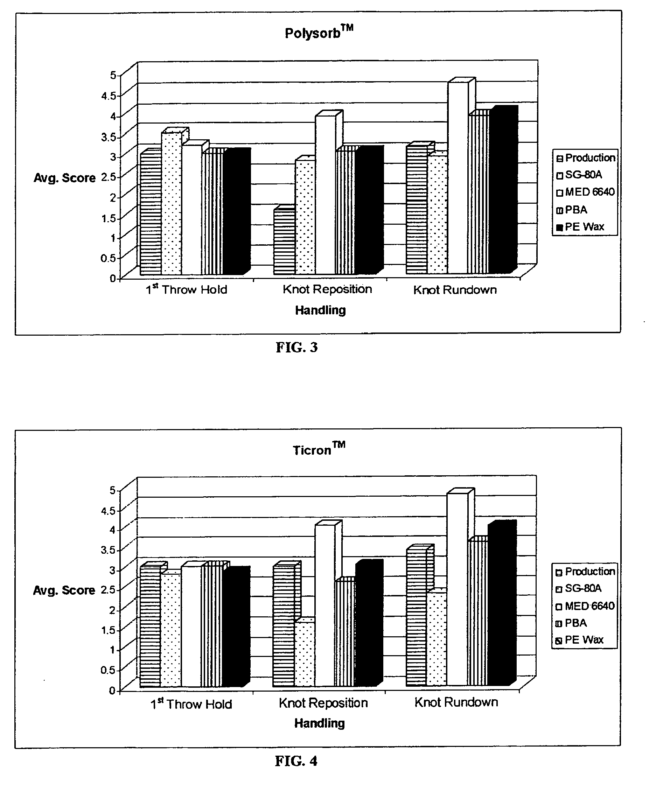

Biodegradable and non-biodegradable sutures coated with MED-6640 2-part silicone, PE wax, PBA, and SG-80A, were tested for knot handling, knot repositioning, and knot rundown. Results for these sutures as well as uncoated and production biodegradable sutures (POLYSORB ™**)** are displayed graphically in FIG. 3. Results for production non-bioabsorbable sutures (TICRON™) are displayed graphically in FIG. 4. Five sutures of each type were tested. The MED-6640 2-part silicone produced the best results for handling of both the biodegradable and non-biodegradable sutures with a knot repositioning score of 3.9 and a knot rundown score of 4.7. The biodegradable sutures coated with SG-80A had a score of 3.5 for knot handling, 2.8 for knot repositioning, and 2.9 for knot rundown, while the SG-80A on Ticron ™ did not perform as well.

### EXAMPLE 4

SEM (scanning electron microscope) images were taken of bioabsorbable yarns coated with the SG-80A (from THERMEDICS™), aliphatic polyether TPU. The coatings covered the external surfaces of the yarns as well as the interstitial areas in the suture. The coating wet the surface of the bioabsorbable yarn, resulting in a thorough coating.

While the above description contains many specifics, these specifics should not be construed as limitations on the scope of the present disclosure, but merely as exemplifications of embodiments thereof. Those skilled in the art will envision many other possible variations that are within the scope and spirit of the present disclosure.

## Claims

1. A coated medical device comprising:
at least one non-bioabsorbable filament comprising a polymer comprising a polyester; and
a coating comprising a two part silicone on at least a portion of a surface of the non-bioabsorbable filament,
wherein the two part silicone is at a concentration of from about 1% to about 30% by weight in solution.

2. The coated medical device of claim 1, wherein the non-bioabsorbable filament comprises polyethylene terephthalate.

3. The coated medical device of claim 1 or claim 2, wherein the medical device comprises a multi-filament suture.

4. The coated medical device of any of claims 1 to 3, wherein the coated medical device has an average fatigue of from about 20 to about 230 cycles to failure.

5. The coated medical device of any preceding claim, wherein the solution comprises a solvent selected from the group consisting of hexane, xylene, methylene chloride, tetrahydrofuran, chloroform, methylethyl ketone, and combinations thereof.

6. A coated medical device comprising:
at least one non-bioabsorbable filament comprising a polymer comprising a polyester; and
a coating comprising a polybutylene adipate on at least a portion of a surface of the non-bioabsorbable filament,
wherein the polybutylene adipate is at a concentration of from about 1% to about 10% by weight in solution.

7. The coated medical device of claim 6, wherein the coated medical device has an average fatigue of from about 74 cycles to failure to about 236 cycles to failure.

8. The coated medical device of claim 6 or claim 7, wherein the non-bioabsorbable filament comprises polyethylene terephthalate.

9. The coated medical device of any of claims 6 to 8, wherein the medical device comprises a multi-filament suture.

10. The coated medical device of any of claims 6 to 9, wherein the solution comprises a solvent selected from the group consisting of hexane, xylene, methylene chloride, tetrahydrofuran, chloroform, methylethyl ketone, and combinations thereof.

11. A coated medical device comprising:
at least one non-bioabsorbable filament comprising a polymer comprising a polyester; and
a coating comprising a polyethylene wax on at least a portion of a surface of the non-bioabsorbable filament,
wherein the polyethylene wax is at a concentration of from about 10% to about 30% by weight in solution.

12. The coated medical device of claim 11, wherein the non-bioabsorbable filament comprises polyethylene terephthalate.

13. The coated medical device of claim 11 or claim 12, wherein the medical device comprises a multi-filament suture.

14. The coated medical device of any of claims 11 to 13, wherein the coated medical device has an average fatigue of from about 67 cycles to failure to about 202 cycles to failure.

15. The coated medical device of any of claims 11 to 14, wherein the solution comprises a solvent selected from the group consisting of hexane, xylene, methylene chloride, tetrahydrofuran, chloroform, methylethyl ketone, and combinations thereof..
